# FASCICULE DE BREVET EUROPEEN

(11) **EP 1 141 548 B1**
(45) Date de publication et mention de la délivrance du brevet: **08.10.2003**
(21) Numéro de dépôt: 99959500.2
(22) Date de dépôt: 20.12.1999
(51) Int. Cl.: F04B 45/073, F04B 45/08

(54) **COMPRESSEURS PERISTALTIQUES ADAPTES A LA COMPRESSION NON RELAXANTE DE GAZ POLARISE**
PERISTALTISCHE KOMPRESSOREN ZUR NICHT-RELAXIERENDEN VERDICHTUNG POLARISIERTER GASE
PERISTALTIC COMPRESSORS ADAPTED TO NON-RELAXING COMPRESSION OF POLARISED GAS

(30) Priorité: 23.12.1998 FR 9816366
(43) Date de publication de la demande: 10.10.2001
(73) Titulaire: CENTRE NATIONAL DE LA RECHERCHE SCIENTIFIQUE, 75016 Paris Cédex (FR)
(72) Inventeur: NACHER, Pierre-Jean, F-94200 Ivry sur Seine (FR)
(74) Mandataire: Breese, Pierre
(86) Numéro de dépôt international: FR9903205
(87) Numéro de publication internationale: WO00039464

(56) Documents cités:
- EP-A- 0 869 283
- DE-A- 3 703 124
- FR-A- 1 394 047
- FR-A- 1 431 105
- FR-A- 2 744 932
- NL-C- 21 604
- US-A- 5 049 048
- US-A- 5 342 182
- US-A- 5 545 396
- ROSEN, M. S. ET AL: "Polarized 129Xe optical pumping/spin exchange and delivery system for magnetic resonance spectroscopy and imaging studies" REVIEW OF SCIENTIFIC INSTRUMENTS, [Online] vol. 70, no. 2, février 1999 (1999-02), pages 1546-1552, XP002131321 Retrieved from the Internet: <URL:http://www.hyperfine-research.com/> [retrieved on 2000-02-23]

## Description

La présente invention concerne le domaine des compresseurs péristaltiques. Ce type de compresseur utilise le principe des pompes péristaltiques, où un ensemble de galets pressent un tuyau souple, et font progresser lors de leur rotation autour d'un axe le fluide que ce tuyau contient.

Les compresseurs péristaltiques de l'art antérieur fonctionnent principalement en pression. En dépression, le tuyau dans lequel circule le fluide à tendance à s'écraser, ce qui empêche le fonctionnement de la pompe. A titre d'exemple de pompe péristaltique connue dans l'art antérieur, le brevet FR2640698 décrit une pompe péristaltique comportant plusieurs vannes à manchon à pincement par fluide sous pression. Son corps est composé d'au moins deux modules disposés en série et comportant chacun un manchon. les extrémités de chaque manchon sont solidarisées de façon étanche aux bords de l'ouverture d'aspiration et de refoulement de chaque module. Le module situé du coté de l'alimentation de la pompe joue le rôle de clapet d'aspiration. Le module situé du côté de la sortie de la pompa jous le rôle de clapet, de refoulement.

Le brevet européen EP869283 concerne une pompe péristaltique comportant un rotor ayant de préférence deux ou trois galets qui roulent contre au moins un tuyau contenant le liquide à pomper. La compression du tuyau presse les galets contre la surface périphérique circulaire d'un stator central vibrant en forme de disque ou d'anneau, qui guide les galets et les fait tourner. Le stator est mis en vibration par des moyans piézo-électriques et vibre par extension dans son plan radial selon une onde progressive. Le châssis du rotor est dépourvu d'arbre central.

Le brevet allemand DS3703124 décrit une pompe à noyau flexible comprenant:
- a) une enveloppe comportant :
   - a1) une tubulure d'aspiration et une tubulure de compression,
   - a2) une paroi périphérique qui, au moins dans une zone d'écrasement située le long de la longue pièce de liaison comprise entre la tubulure d'aspiration et la tubulure de compression, à la forme d'un cylindre de section circulaire et
   - a3) une paroi avant plane et une paroi arrière plane,
- b) un tuyau flexible qui va de la tubulure d'aspiration à la tubulure de compression le long de la paroi périphérique dans la zone d'écrasement et dont la périphérie correspond essentiellement au double de la distance comprise entre la paroi avant et la paroi arrière,
- c) un rotor dont l'axe de rotation coïncide avec l'axe de la zone d'écrasement de la paroi périphérique en forme de cylindre de section circulaire, et qui comporte au moins deux corps d'écrasement disposés symétriquement par rapport a un point et qui tourne de telle manière que chaque corps d'écrasement écrase le tuyau flexible depuis la zone de la tubulure d'aspiration jusqu'à la zone de la tubulure de compression.

Un dispositif de séparation isole hermétiquement une zone d'aspiration, c'est-a-dire une partie de l'intérieur de la pompe dans laquelle une dépression règne pendant le pompage, d'une zone de compression dans laquelle ne règne aucune dépression.

Le dispositif de séparation comporte un dispositif assurant l'étancheité au niveau du rotor et un joint elastique. Le dispositif assurant l'étanchéité au niveau du rotor est solidaire du rotor et sépare hermétiquement celui-ci de la paroi avant et de la paroi arrière.

Le brevet américain US5049048 décrit une pompe tubulaire comporte un carter avec un raccord foulant et un raccord aspirant. Un premier tuyau est relié aux raccords foulant et aspirant et agencé contre la paroi intérieure du carter. Des éléments de compression peuvent se déplacer le long du tuyau de telle manière que ledit tuyau peut être comprimé par chacun des éléments de compression de la direction du raccord aspirant vers la direction du raccord foulant. La pompe comporte au moins un second tuyau élastiquement déformable dont l'une des extrémités se termine dans la partie intérieure du carter tandis que son autre extrémité se termine à l'extérieur dudit carter.

Le brevet américain US 5 261 793 concerne un compresseur permettant d'atteindre de très basses pressions par le choix d'un tube adapté (diamètre assez petit, paroi assez épaisse) pour éviter son aplatissement (col 5, lignes 10-12). Cette contrainte sur le choix du tube est a priori gênante pour l'application au gaz polarisé : une faible section augmente le rapport surface/volume est augmente la relaxation pour le gaz polarisé; une faible section diminue le débit de pompe toutes choses égales par ailleurs. En outre, il importe de pouvoir choisir le type de matériau pour le tube en fonction de ses propriétés de compatibilité avec l'application au gaz polarisé, sans être restreint aux tubes les plus rigides. Le brevet FR 1354047 A, décrit un compresseur dans lequel apparaissent à la fois la mise sous vide du corps de pompe et l'entraînement par moteur péristaltique. Par contre, il ne s'agit pas d'une pompe péristaltique, puisque la fluide n'est pas contenu dans un tube, mais dans un compartiment déformable fermé par des membranes en caoutchouc. Un tel compresseur est totalement inadapté à la réalisation d'un compresseur pour gaz polarisé.

Le but de l'invention est de remédier à un premier inconvénient des pompes péristaltiques de l'art antérieur résultant de la présence de pièces métalliques, notamment de pièces magnétiques. Ces pièces magnétiques empêchent l'utilisation pour la compression et le pompage de gaz polarisés. Pour remédier à cet inconvénient, l'invention propose de mettre à profit la réversibilité et l'effet moteur d'un dispositif péristaltique afin de ne pas nécessiter de moteur externe d'entraînement des galets presseurs. Ceci permet en particulier d'avoir un dispositif totalement amagnétique et sans alimentation électrique, ce qui est important pour la compression sans relaxation de gaz polarisé, en particulier au sein d'un appareil d'imagerie RMN, ou dans une région de champ magnétique intense, etc...

L'invention vise également à éviter l'inconvénient des pompes de l'état de la technique dont le fonctionnement ne permet pas d'atteindre des niveaux de dépression importants, en raison de l'écrasement des tuyaux. Lorsque les tuyaux contiennent un fluide à une pression inférieure à la pression atmosphérique, ils conservent la déformation occasionnée par les galets du rotor, et ne reprennent pas leur volume initial. Pour remédier à cet inconvénient, l'invention concerne dans son acception la plus générale un compresseur péristaltique conforme à la revendication principale.

L'invention concerne selon une variante avantageuse une pompe péristaltique comportant un rotor entraîné par un moteur muni d'une pluralité de galets, et au moins un tuyau caractérisé en ce que le moteur est constitué par au moins un tuyau alimenté par un fluide sous pression, entraînant en rotation un rotor péristaltique muni de galets accouplé au rotor de pompage.

Avantageusement, le rotor d'entraînement et le rotor de pompage forment une pièce unique.

Selon un mode de réalisation préféré, le dispositif objet de l'invention comporte une alternance de tuyaux pour le pompage et la compression, et de tuyaux pour l'entraînement du moteur.

Selon un mode de mise en oeuvre préféré, l'invention concerne un compresseur péristaltique adapté à la compression non relaxante de gaz polarisé comportant uniquement des pièces amagnétiques.

Selon une variante avantageuse, le rotor comprend deux ou trois galets libres en rotation.

De préférence, les tuyaux moteurs et de pompage sont décalés angulairement.

Selon une variante particulière, le dispositif comprend un tuyau central pour le pompage, et deux tuyaux latéraux pour l'entraînement du rotor, disposés de part et d'autre du tuyau central.

De préférence, le ou les tuyaux moteur reçoivent un fluide sous une pression comprise entre 1 et 5 bars.

selon une variante préférée, le rotor et les tuyaux de pompage sont placés dans une enceinte dépressurisée à l'intérieur de laquelle règne une pression inférieure à la pression atmosphérique.

Avantageusement, l'enceinte dépressurisés est reliée à une source de dépression.

La présente invention résout en particulier le problème de la compression efficace par un dispositif simple d'un gaz (ou d'un mélange gazeux) fortement polarisé, et cela sans perte significative de polarisation. Ce problème est rencontré lors de la préparation d'hélium-3 polarisé par pompage optique à 1083 nm, procédé qui n'est efficace qu'à des pressions de gaz inférieures à la pression atmosphérique, pour toutes applications où une pression plus importante est requise. C'est ainsi que, suivant le procédé employé, le gaz polarisé aura une pression de l'ordre du millibar, ou bien de quelques dizaines à centaines de millibars, alors que des applications telles que l'imagerie in vivo de volumes aériens requièrent du gaz à un bar ou plus. Ce problème peut aussi être rencontré pour du xénon polarisé par pompage optique, ou plus généralement pour tout gaz ou mélange gazeux dont on souhaite forcer le transfert et/ou l'accumulation dans un volume donné, sans que soit significativement affectée la polarisation nucléaire hors d'équilibre (l'hyperpolarisation) de ce gaz.

Les techniques utilisées à ce jour pour remplir cette fonction reposent sur des dispositifs cryogéniques ou mécaniques. Les dispositif cryogéniques, qui stilisent un cycle avec changement de phase pour le gaz polarisé (liquéfaction de l'hélium, liquéfaction ou solidification du xénon), ne sont pas applicables à tous les mélanges gazeux et ne sont pas simples à mettre en oeuvre dans tous les environnements. Les dispositifs compresseurs mécaniques usuels (pompes à piston, à membrane, à palettes...) ont l'inconvénient de ne permettre qu'un taux de compression limité (et donc de requérir en pratique plusieurs étages) et d'être très délicats à adapter aux exigences rigoureuses d'amagnétisme qu'impose la compression sans relaxation de gaz polarisé. Il existe en outre un problème supplémentaire pour l'entraînement mécanique d'un compresseur, qui ne paut être assuré par un moteur électrique (générateur de champs magnétiques provoquant une perte rapide de polarisation du gaz) disposé à proximité du compresseur.

Une pompe péristaltique présente pour les gaz polarisés un certain nombre d'avantages par rapport aux dispositifs cryogéniques ou mécaniques. Elle autorise a priori un débit volumique donné sans limite théorique au taux de compression, et est aisément réalisable avec des matériaux amagnétiques, voire non métalliques. Elle est donc susceptible de fonctionner dans tout environnement (y compris en champ magnétique intense), et sans induire de relaxation nucléaire notable. En revanche, elle est à priori impropre à assurer l'écoulement et la compression d'un fluide à pression notablement inférieure à la pression atmosphérique (cette pression aplatit les tuyaux souples, et empêche en pratique tout débit de fluide à pression inférieure à 0.7 ou 0.8 bar absolus) ; en outre, le problème de son entraînement mécanique est identique à celui évoqué plus haut pour les autres types de compresseurs mécaniques.

Les deux améliorations présentées dans le principe général de l'invention, et détaillées ci-après, permettent à une pompe péristaltique de finalement résoudre ces derniers problèmes, qui sont en particulier rencontrés pour la compression ou la manipulation de gaz hyperpolarisé dans des environnements très contraignants.

Il sera décrit ici un dispositif compresseur très simple et adapté à la compression non relaxante de gaz polarisé (c'est à dire sans induire de perte significative de polarisation). Ce dispositif est en particulier bien adapté à la compression d'hélium produit selon les modalités faisant l'objet du brevet FR2744932 ayant pour objet un procédé permettant la polarisation par pompage optique d'un gaz d'hélium à forte pression. Il peut être également employé pour la compression non relaxante de gaz polarisé produit à plus basse pression par des techniques conventionnelles, ou bien encore pour transférer un gaz polarisé d'un récipient à un autre.

Une application importante du dispositif concerne la compression d'un gaz d'hélium polarisé par pompage optique. On trouvera dans le texte du brevet déjà cité référence à la technique de polarisation traditionnelle par pompage optique effectué sur la transition atomique de longueur d'onde proche de 1083 nm, en présence d'un faible champ magnétique (en général de l'ordre du milli-Tesla) : dans ces conditions, le pompage optique n'est efficace que lorsque la pression du gaz est de l'ordre du millibar (1 mbar = 100 Pa) . Ce même texte décrit comment l'utilisation judicieuse d'un champ magnétique assez intense permet d'effectuer un pompage optique efficace dans une large gamme de pressions du gaz, qui restent cependant inférieures à la pression atmosphérique (couramment 10 à 100 mbar). Pour certaines applications, ce gaz doit être porté à la pression atmosphérique (pur, ou mélangé avec un autre gaz) avant de pouvoir être utilisé ; par exemple, pour l'imagerie de résonance magnétique in-vivo, le gaz doit pouvoir être inhalé par un sujet. Pcur le stockage ou le transport, le gaz pourra avantageusement être porté à une pression de plusieurs bars dans des récipients adaptés ; il en sera finalement extrait pour l'utilisation finale souhaité.

Le pompage optique d'hélium a 1083 nm permet de polariser les noyaux du gaz à un rythme dépendant de nombreux paramètres, dont la puissance lumineuse disponible, et pouvant Varier de 10¹⁷ à 10¹⁹ atomes par seconds. En régime continu, cela correspond à des débits volumiques de gaz inversement proportionnels à la pression P, dont des exemples sont donnés dans le tableau ci-dessous ;

| Rythme de polarisation (atomes/s) | Puissance laser typique (à 1083 nm) | Débit à p=1mbar *champ magnétique faible* | Débit à P=30mbar *champ magnétique fort* |
|---|---|---|---|
| 10¹⁷ | 50 mW | 0.22 l/min | 7 cm³/min |
| 10¹⁹ | 5 W | 22 l/min | 0.7 l/min |

Ces ordres de grandeur indiquent clairement les caractéristiques de débit qui seront requises d'un dispositif compresseur, suivant la source laser employée et la pression à laquelle le pompage optique est effectué. Le débit volumique d'une pompe péristaltique est de produit de la section interne du tube employé par la vitesse linéaire des galets presseurs. Pour le dispositif prototype que nous avons réalisé, cette section est de 0.4 cm², et une rotation à 1200 tours/min entraîne un débit (par tube) de l'ordre de 14 l/min. De telles caractéristiques sont tout à fait adaptées aux débits typiques requis dans diverses situations, plusieurs tubes en parallèle (ou un tube de section supérieure) permettant un débit plus grand à vitesse de rotation donnée.

Il n'y a pas de limite de principe au taux de compression atteint par une pompe péristaltique. En pratique, la pression maximale atteinte est limitée par la résistance à la surpression du tube employé (souvent 2 à 3 bars). La pression minimale à laquelle une pompe fonctionne est classiquement limitée par l'aptitude du tube à reprendre sa forme (section circulaire) après le passage d'un galet presseur. Son élasticité est en général insuffisante en présence d'une surpression extérieure, qui le maintient aplati. Selon une des caractéristiques de l'invention, c'est-à-dire le fonctionnement dans une enceinte évacuée, la pression interne au tube est toujours supérieure à celle de l'enceinte où il est plongé, et le débit nominal demeure jusqu'à pression nulle. En conséquence, un simple étage de pompe péristaltique fonctionnant dans une enceinte évacuée est adapté à la compression de gaz depuis une pression adaptée au pompage optique de l'hélium jusqu'à une pression pouvant par exemple atteindre 2 bars. Dans le cas où il est requis d'atteindre des pressions plus importantes, un deuxième étage de compression pourra être utilisé ; il sera même possible d'utiliser à cet usage une pompe péristaltique fonctionnant dans une enceinte pressurisée afin de ne plus être limité par la résistance à la surpression du tube employé.

Pour toute application où l'on manipule du gaz polarisé, il convient d'utiliser des dispositifs n'induisant pas d'inhomogénéités de champ magnétique au niveau du gaz.

De telles insomogénéités sont en effet susceptibles d'induire une rapide relaxation nucléaire vers une valeur quasi-nulle de la polarisation. Ces inhomogrénéités peuvent être à grande échelle et concerner le volume du gaz. Il convient, pour les éviter, de ne pas utiliser des matériaux présentant une susceptibilité magnétique significative, et cela d'autant plus qu'ils sont plus près du gaz polarisé. Les matériaux ferromagnétiques sent bien sûr à proscrire, mais il faut également éviter de ne pas utiliser un grand nombre d'alliages couramment employés pour leurs propriétés mécaniques (aciers inoxydables, laiton, bronze,...). L'utilisation de moteurs électriques, générateurs de champs magnétiques très inhomogènes, est en général impossible à des distances inférieures à un ou quelques mètres. Enfin, il peut être avantageux, par exemple en présence de champs électromagnétiques intenses tels qu'on en rencontre au sein des appareils d'IRM, de proscrire toute pièce métallique conductrice. Mous verrons sur l'exemple du dispositif réalisé que ces contraintes ont pu être respectées sans inconvénient.

Ces inhomogénéités de champ magnétique peuvent aussi être induites à une échelle microscopique par les caractéristiques de surface des matériaux directement au contact du gaz polarisé. De plus, aucune contamination du gaz polarisé, chimique ou biologique, ne doit résulter du contact du gaz avec ces matériaux ; ceci est particulièrement important pour les applications où le gaz est ensuite inhalé, mais la pureté du gaz est également essentielle lors du processus de polarisation par pompage optique (aucune impureté ne doit pouvoir diffuser du dispositif compresseur vers le volume de pompage optique), et peut jouer un rôle dans la relaxation nucléaire du gaz lors de son transport ou stockage ultérieur. Il convient pour ces différent points de prendre toutes précautions lors du choix des matériaux entrant au contact direct du gaz, et d'utiliser des configurations propres à éviter toute contamination du gaz dans le volume de pompage pour le dispositif réalisé.

Un des point évoqués ci-dessus - la non contamination du gaz en amont du compresseur par les impuretés que celui-ci introduit inévitablement - impose de concevoir un dispositif à écoulement monotone, sans refoulement. Dans une pompe péristaltique, la rotation des galets entraîne le fluide dans le tube de facon monotone et régulière dans le sens de la rotation. Néanmoins, lorsqu'un galet entre en contact avec le tube et le comprime, il provoque une diminution du volume accessible au fluide à l'entrée de la pompe, ce qui tend à refouler du fluide. La compétition de ce deux effets pourra éventuellement entraîner un refoulement net de fluide pour certaines positions des galets, suivant le rapport des diamètres respectifs des galets et du chemin de roulement où est pressé le tuyau (le bilan net de ces effets dépend également de la façon précise dont le tube est progressivement écrasé).

Un dispositif péristaltique, s'il est en pratique toujours employé pour faire débiter ou pour comprimer un fluide, peut également être utilisé comme moteur : lorsqu'une différence de pression est appliquée au fluide contenu dans le tube, un couple moteur s'applique aux galets. Ce couple peut directement être déduit par dérivation de l'effet de la rotation sur le débit. Dans la plupart des réalisations commerciales de pompes péristaltiques, les caractéristiques géométriques sont telles qu'un refoulement du fluide intervient plusieurs fois par tour ; dans ce cas, le couple moteur change de signe lors de la rotation, et une rotation continue n'est pas possible. En revanche, lorsque les caractéristiques géométriques sont adéquates, le couple moteur a un signe constant et une rotation continue devient possible sous réserve que la dissipation par les frottements n'excède pas la puissance motrice. Dans le cas du dispositif réalisé, une large plage de fonctionnement du moteur a pu être obtenue.

La pompe péristaltique adaptés à la compression de gaz polarisé comporte un ou des tuyaux placés à l'intérieur d'un volume essentiellement cylindrique, servant comme il est traditionnel de surface d'appui aux tuyaux, mais construit de façon à être étanche vis à vis de l'air environnant, Suivant la configuration retenue, des traversées étanches permettent l'entrée et la sortie des fluides circulant dans les tuyaux. Un rotor comportant au moins deux galets, par exemple trois dans l'exemple décrit, libres de tourner sur leur axe est disposé de façon à convenablement comprimer le ou les tuyaux.

Pour un fonctionnement totalement pneumatique, trois tuyaux sont utilisés : deux d'entre eux sont soumis à une pression ajustable d'air ou d'eau sous pression, et le couple résultant suffit à entraîner le rotor. Le troisième tuyau est alors utilisable pour faire circuler ou comprimer un fluide, par exemple du gaz polarisé. Pour un fonctionnement en simple pompe, un axe tournant traversant de façon étanche la paroi de la pompe est utilisé pour entraîner mécaniquement le rotor. Dans les deux cas, la pression du volume interne du dispositif peut être ajustée, et en particulier rendue assez basse pour permettre le pompage de fluides à pression arbitrairement basse, selon l'une des variantes de l'invention. ce dispositif fonctionne de façon satisfaisante, et tous les matériaux et pièces qu'ils comporte ne provoquent aucune relaxation magnétique dans leur voisinage immédiat. En fonction de ces résultats, des dispositifs spécifiques seront développés pour des utilisations particulières. Suivant le rythme de production permis par le laser source, et suivant le type d'utilisation envisagé, il pourra en particulier être plus avantageux de faire usage d'un moteur péristaltique ou d'utiliser un moteur conventionnel.

Pour la plupart des applications, le gaz (hélium ou xénon) hyperpolarisé doit être produit ou porté à une pression de l'ordre de la pression atmosphérique. Actuellement, deux types de méthodes sont utilisées pour produire du gaz polarisé à force polarisation :
la polarisation de l'hélium-3 ou du xénon par échange de spin avec un gaz d'atomes alcalins orientés par pompage optique (par exemple une vapeur de rubidium). Dans le cas des atomes alcalins, le pompage opère sur l'état fondamental des atomes ; aucune décharge n'étant nécessaire dans le gaz, le pompage peut en principe opérer à toute pression. Malheureusement cette méthode est intrinsèquement très lente, car les collisions d'échange qui assurent le transfert de polarisation de l'alcalin au gaz rare sont peu efficaces et constituent un facteur limitant incontournable. Il faut ainsi par exemple 4 à 8 heures pour polariser 100 cm³ d'hélium à une pression de 3.5 à 8 bar avec une polarisation de 5 à 20%. Dans le cas du xénon, il est bien plus favorable de travailler avec une faible pression de xénon, et son accumulation se fait au moyen d'une technique cryogénique.

La polarisation par pompage optique à 1083 nm, avec une décharge dans un gaz à basse pression, suivie d'une compression du gaz polarisé. Si le pompage optique constitue une étape très efficace, la compression fait appel à des techniques à ce jour complexes, qui n'ont été développées que dans peu de laboratoires. Une méthode consiste à utiliser un compresseur mécanique à pistons mobiles, spécialement conçu pour préserver la polarisation et la pureté du gaz.

Une seconde méthode, mis au point par le demandeur et décrite dans le brevet FR2744932 utilise une technique cryogénique de compression. Un prototype de dispositif plus simple, basé sur un modèle commercial de pompe à membrane profondément modifiée pour réduire le caractère relaxant des matériaux habituellement employés, a également été développé au NIST (Washington, USA) et est maintenant utilisé pour des essais d'imagerie avec de l'hélium hyperpolarisé. Signalons enfin que la difficulté technique de compression mécanique peut être fortement réduite en effectuant le pompage optique à une pression plus élevée, ce qui réduit le taux de compression et le débit volumique requis pour le dispositif compresseur.

Mentionnons pour mémoire que l'hélium-3 peut aussi être substantiellement polarisé de manière spontanée, mais dans un très fort champ magnétique et à très basse température. Divers systèmes peuvent être utilisés (fusion de solide polarisé à l'équilibre thermique, refroidissement extrême de mélanges isotopiques très dilués, surpolarisation par pseudo-distillation de mélanges liquides démixés, ...), mais ils requièrent des techniques très sophistiquées et n'ont que tout récemment été utilisés pour produire du gaz polarisé (à Grenoble) avec une polarisation très modeste (de l'ordre du pourtant).

un dispositif compresseur péristaltique intégrant les innovations introduites permet une compression de gaz hyperpolarisé compatible avec l'emploi des diverses techniques de pompage optique, et présente sur les autres dispositifs de compression un certain nombre d'avantages :

Par rapport aux autres méthodes de compression mécanique, la compression péristaltique présente l'avantage de principe de n'avoir pas de limite au taux de compression susceptible d'être obtenu (en un seul étage), et de ne pas nécessiter de pièces mobiles (clapets, soupapes, vannes...) au contact du gaz polarisé, dont l'expérience montre qu'il est très délicat de les réaliser sans qu'elles introduisent une relaxation importante. Les innovations introduites permettent à de tels dispositifs de pomper un fluide à pression arbitrairement basse, et de s'affranchir le cas échéant du problème de l'entraînement mécanique de la pompe. On sait donc maintenant fabriquer un dispositif très simple, en matériaux courants et sans inconvénient de relaxation, qui permet de comprimer et/ou faire écouler du gaz polarisé. La simplicité de réalisation et la compacité de tels dispositifs permettent une réalisation à coût très modeste.

Par rapport aux méthodes cryogéniques, la simplicité, portabilité et facilité d'utilisation du dispositif le feront en général préférer.

Un système de production d'hélium, hyperpolarisé utilisant un tel dispositif serait donc suffisamment simple pour être opérable hors d'un laboratoire, et par exemple portable ou implantable sur le site d'utilisation. Outre son utilité essentielle pour la production de gaz par pompage optique, ce dispositif est également susceptible d'être intégré à des systèmes contrôlant la circulation de gaz polarisé entre récipients, ou même l'administration contrôlée de gaz polarisé aux patients ou sujets examinés en IRM.

## Revendications

1. Compresseur péristaltique adapté à la compression non relaxante de gaz polarisé comportant un rotor entraîné par un moteur muni d'une pluralité de galets, et au moins un tuyau, le rotor et les tuyaux de pompage étant placés dans une enceinte dépressurisée à l'intérieur de laquelle règne une pression inférieure à la pression atmosphérique, comportant uniquement des pièces amagnétiques, **caractérisé en ce que** le moteur est constitué par au moins un tuyau alimenté par un fluide sous pression, entraînant en rotation un rotor péristaltique muni de galets accouplé au rotor de pompage.

2. Compresseur péristaltique selon la revendication 1 **caractérisé en ce qu'**il comporte une alternance de tuyaux pour le pompage et la compression, et de tuyaux pour l'entraînement du moteur.

3. Compresseur péristaltique selon la revendication 2 **caractérisé en ce que** le compresseur comporte uniquement des pièces amagnétiques

4. Compresseur péristaltique selon l'une quelconque des revendications précédentes **caractérisé en ce que** le rotor comprend deux ou trois galets libres en rotation.

5. Compresseur péristaltique selon l'une quelconque des revendications précédentes **caractérisé en ce que** les tuyaux de pompage et moteur sont décalés angulairement.

6. Compresseur péristaltique selon l'une quelconque des revendications précédentes **caractérisé en ce qu'**il comprend un tuyau central pour le pompage, et deux tuyaux latéraux pour l'entraînement du rotor, disposés de part et d'autre du tuyau central.

7. Compresseur péristaltique selon l'une quelconque des revendications précédentes **caractérisé en ce que** le tuyau moteur reçoit un fluide sous une pression de 1 à 5 bars.

8. Compresseur péristaltique selon l'une quelconque des revendications précédentes **caractérisé en ce que** le rotor d'entraînement et le rotor de pompage forment une pièce unique.

9. Compresseur péristaltique selon la revendication 8 **caractérisé en ce que** l'enceinte dépressurisée est reliée à une source de dépression.

10. Equipement de production d'hélium hyperpolarisé par pompage optique **caractérisé en ce qu'**il comprend un compresseur péristaltique étant réalisé uniquement des pièces amagnétiques et comportant un rotor entraîné par un moteur muni d'une pluralité de galets, et au moins un tuyau, le rotor et les tuyaux de pompage étant placés dans une enceinte dépressurisée à l'intérieur de laquelle règne une pression inférieure à la pression atmosphérique.

11. Équipement de production d'hélium hyperpolarisé par pompage optique selon la revendication 10 **caractérisé en ce qu'**il comporte des moyens pour l'administration contrôlée de gaz polarisé aux patients ou sujets examinés en IRM.

## Patentansprüche

1. Peristaltischer Verdichter, geeignet zur nichtabklingenden Verdichtung von polarisiertem Gas mit einem Läufer, welcher durch einen mit einer Vielzahl von Geröllen versehenen Motor angetrieben wird, und mindestens einem Schlauch, wobei der Läufer und die Schläuche in einem Raum mit vermindertem Luftdruck angeordnet sind, in welchem ein Luftdruck herrscht, welcher niedriger ist als der atmosphärische Luftdruck, mit nur unmagnetischen Teilen, **dadurch gekennzeichnet, dass** der Motor aus mindestens einem mit einer unter Druck gesetzten Flüssigkeit versorgten Schlauch besteht, welcher einen peristaltischen Läufer in Drehung setzt, der mit an den Pumpläufer gekuppelten Geröllen versehen ist.

2. Peristaltischer Verdichter nach Anspruch 1, **dadurch gekennzeichnet, dass** er eine Aufeinanderfolge von Schläuchen zum Pumpen und zur Verdichtung und Schläuchen zum Antrieb des Motors aufweist.

3. Peristaltischer Verdichter nach Anspruch 2, **dadurch gekennzeichnet, dass** der Verdichter nur unmagnetische Teile aufweist.

4. Peristaltischer Verdichter nach einem der vorangehenden Ansprüche, **dadurch gekennzeichnet, dass** der Läufer zwei oder drei drehbare Gerölle aufweist.

5. Peristaltischer Verdichter nach einem der vorangehenden Ansprüche, **dadurch gekennzeichnet, dass** die Pumpschläuche und der Motor winkelmäßig versetzt sind.

6. Peristaltischer Verdichter nach einem der vorangehenden Ansprüche, **dadurch gekennzeichnet, dass** er einen zentralen Schlauch für das Pumpen und zwei seitliche Schläuche für den Antrieb des Läufers aufweist, welche beidseitig des zentralen Schlauches angeordnet sind.

7. Peristaltischer Verdichter nach einem der vorangehenden Ansprüche, **dadurch gekennzeichnet, dass** der Motorschlauch eine Flüssigkeit unter einem Druck von 1 bis 5 bar aufnimmt.

8. Peristaltischer Verdichter nach einem der vorangehenden Ansprüche, **dadurch gekennzeichnet, dass** der Antriebsläufer und der Pumpläufer ein einziges Teil bilden.

9. Peristaltischer Verdichter nach Anspruch 8, **dadurch gekennzeichnet, dass** der Raum mit vermindertem Luftdruck an eine Unterdruckquelle angeschlossen ist.

10. Ausrüstung zur Herstellung von hyperpolarisiertem Helium durch optisches Pumpen, **dadurch gekennzeichnet, dass** sie einen peristaltischen Verdichter umfasst, welcher nur aus unmagnetischen Teilen besteht und einen Läufer aufweist, welcher von einem mit einer Vielzahl von Geröllen versehenen Motor angetrieben wird, und mindestens einen Schlauch, wobei der Läufer und die Pumpschläuche in einem Raum mit vermindertem Luftdruck angeordnet sind, in welchem ein Luftdruck herrscht, der niedriger ist als der atmosphärische Luftdruck.

11. Ausrüstung zur Herstellung von hyperpolarisiertem Helium durch optisches Pumpen nach Anspruch 10, **dadurch gekennzeichnet, dass** sie Mittel aufweist zur kontrollierten Verabreichung des polarisierten Gases an Patienten oder Personen, die mit einer MRI untersucht werden.

## Claims

1. Peristaltic compressor adapted to non relaxing compression of polarised gas comprising a rotor driven by a motor provided with a plurality of rollers and at least one pipe, the rotor and the pumping pipes being placed inside a depressurised containment inside which the pressure is less than atmospheric pressure, comprising non-magnetic parts only, **characterised in that** the motor is composed of a pipe supplied with a pressurised fluid causing a rotational movement of a peristaltic rotor fitted with rollers coupled to the pumping rotor.

2. Peristaltic compressor according to claim 1, **characterised in that** it comprises alternating pipes for pumping and compression, and pipes for driving the motor.

3. Peristaltic compressor according to claim 2, **characterised in that** the compressor comprises non-magnetic parts only.

4. Peristaltic compressor according to any one of the previous claims, **characterised in that** the rotor comprises two or three rollers free in rotation.

5. Peristaltic compressor according to any one of the previous claims, **characterised in that** the pumping and driving pipes are offset from each other at different angles.

6. Peristaltic compressor according to any one of the previous claims, **characterised in that** it comprises a central pipe for pumping, and two lateral pipes for driving the rotor, arranged on each side of the central pipe.

7. Peristaltic compressor according to any one of the previous claims, **characterised in that** the driving pipe receives a fluid at a pressure of 1 to 5 bars.

8. Peristaltic compressor according to any one of the previous claims, **characterised in that** the driving rotor and the pumping rotor form a single part.

9. Peristaltic compressor according to claim 8, **characterised in that** the depressurised containment is connected to a negative pressure source.

10. Helium production equipment hyperpolarised by optical pumping, **characterised in that** it comprises a peristaltic compressor composed of non-magnetic parts only and comprising a rotor driven by a motor provided with a plurality of rollers and at least one pipe, the rotor and the pumping pipes being located in a depressurised containment inside which the pressure is less than atmospheric pressure.

11. Helium production equipment hyperpolarised by optical pumping according to claim 10, **characterised in that** it comprises means for controlled administration of polarised gas to patients or subjects examined in MRI.
